# EUROPEAN PATENT SPECIFICATION

(11) **EP 2 400 866 B1**
(45) Date of publication and mention of the grant of the patent: **08.05.2013**
(21) Application number: 10704931.4
(22) Date of filing: 18.02.2010
(51) Int. Cl.: A41D 13/02, F24F 3/16

(54) **CLEAN ROOM, CLEAN ROOM SUIT AND ARRANGEMENT THEREFOR**
REINRAUM, REINRAUMANZUG UND ANORDNUNG DAFÜR
SALLE BLANCHE, COMBINAISON POUR SALLE BLANCHE ET SYSTÈME ASSOCIÉ

(30) Priority: 26.02.2009 US 155780 P
(43) Date of publication of application: 04.01.2012
(73) Proprietor: Ortner Cleanroom Engineering GmbH, 9500 Villach (AT)
(72) Inventor: ORTNER, Josef, A-9872 Millstatt (AT)
(74) Representative: Dilg, Andreas
(86) International application number: PCT/EP2010/052078
(87) International publication number: WO 2010/097333

(56) References cited:
- US-A- 4 304 224
- US-A- 4 985 933
- US-A- 6 062 976
- US-A1- 2006 258 279
- US-A1- 2008 251 081

## Description

The invention relates to a clean room suit.

Furthermore, the invention relates to a clean room lock.

The invention further relates to a clean room arrangement.

Moreover, the invention relates to a method of managing a transition of a person between an interior and an exterior of a clean room.

A clean room is an environment, typically used in manufacturing or scientific research for instance in the context of semiconductor or pharmaceutical or medical technology, that has a low level of environmental pollutants such as dust, airborne microbes, aerosol particles and chemical vapors. More accurately, a clean room has a controlled level of contamination that is specified by the number of particles per cubic meter at a specified particle size.

A clean room suit is an overall garment worn by a user in a clean room. One common type is an all-in-one coverall worn by semiconductor and nanotechnology line production workers, technicians, and process engineers, as well as people in similar roles creating sterile products for the medical device industry.

However, entry into or exit out of a clean room may be cumbersome for a user and may be time consuming and cost-intensive.

A clean roan suit of the prior art is disclosed in the document US-6,030,976,(DE-GUZMAN)

It is an object of the invention to simplify passage of a person from an unclean environment to a clean room, or vice versa.

In order to achieve the object defined above, a clean room suit, a clean room lock, a clean room arrangement, and a method of managing a transition of a person between an interior and an exterior of a clean room according to the independent claims are provided.

According to an exemplary embodiment of the invention, a clean room suit for clothing a person (for instance in a clean room) is provided, the clean room suit comprising a fabric (for instance designed as an overall) configured to cover at least a part of a (for instance the entire) body of the person, and a clean room lock adapter (particularly an adapter configured for coupling to a tube of the clean room lock) arranged on and/or in (for instance integrated in) the fabric and being configured for coupling to a clean room lock in such a manner that, in an operation mode in which the clean room lock is coupled to the clean room lock adapter and a cleaning procedure is activated (for instance when a cleaning procedure for cleaning the clean room suit is activated), particles (such as dust and/or germs) on and/or in the fabric are moved out (for instance are blown out) of the fabric (for instance from an interior and/or from a surface of the fabric) towards an exterior (for example an outer environment) of the clean room suit.

According to another exemplary embodiment of the invention, a clean room lock for cleaning a person transmitting between an interior and an exterior of a clean room (for instance for cleaning a person passing from an unclean environment towards a clean room, or in the opposite direction) is provided, the clean room lock comprising a clean room suit adapter (particularly an adapter connected to a tube of the clean room lock and to be coupled to a corresponding adapter of the clean room suit) configured for coupling to a clean room suit to clothe the person and configured so that, in an operation mode in which the clean room suit adapter is coupled to the clean room suit and a cleaning procedure is activated (for instance when a cleaning procedure for cleaning the clean room suit is activated), particles (such as dust and/or germs) on and/or in the clean room suit are moved out (for instance are blown out) of the clean room suit (for instance from an interior and/or from a surface of the clean room suit) towards an exterior of the clean room suit.

According to still another exemplary embodiment of the invention, a clean room arrangement is provided which comprises a clean room suit having the above mentioned features, and a clean room lock having the above mentioned features, wherein the clean room suit adapter (of the clean room lock) and the clean room lock adapter (of the clean room suit) are configured (to match to one another) for coupling to one another.

According to yet another exemplary embodiment of the invention, a method of managing a transition of a person in a clean room lock between an interior and an exterior of a clean room (for instance for cleaning a person passing from an unclean environment towards a clean room, or in the opposite direction) is provided, wherein the method comprises clothing the person with a clean room suit having a fabric (for instance designed as an overall) covering at least a part of a (for instance the entire) body of the person, coupling a clean room lock adapter arranged on and/or in the fabric to a clean room suit adapter of the clean room lock, and activating a cleaning procedure controlled by the clean room lock so that particles on and/or in the fabric are moved out of the fabric towards an exterior of the clean room suit (for instance activating a cleaning procedure for cleaning the clean room suit).

In the context of this application, the term "clean room" may particularly denote an area in which air quality, temperature, humidity, and particle concentration may be regulated and monitored in order to protect sensitive equipment from contamination. Air in a clean room may be repeatedly filtered to remove dust particles and other impurities that can damage the production of highly sensitive technologies such as the semiconductor technology. Hence, a clean room may be denoted as a room in which the concentration of air-born particulate matter may be controlled at specific limit to facilitate the manufacture of sterile and highly purified products. In the context of pharmaceutical technology and medical technology, such particles may to be controlled and monitored and germs may be eliminated which might deteriorate the sterile properties of such a room.

The term "clean room lock" may particularly denote a device or chamber permitting the passage of people (and optionally objects) between a clean room ("clean side") and a surrounding ("unclean side"). Such a lock may comprise a small chamber with for instance two airtight doors in series which (in the absence of an emergency case) do not open simultaneously. Hence, a clean room lock can be used to allow passage of persons and objects between a clean environment and a less clean environment. Therefore, within such a lock, cleaning procedures may be performed.

The term "particles" may particularly denote any particulate matter in the air and/or on objects which might deteriorate sterile and clean properties. For instance, such particles may include dust, germs, smoke, etc.

The term "adapter" may particularly denote parts that connect two devices or systems to enable them to work together. Such an adapter can be a device, mechanism or fitting usable to make two parts of different design, shape or size fit to each other. Thus, such an adapter may achieve operative compatibility between devices. For example, a clean room suit adapter may be a fastening element forming part of a clean room lock for fastening to a clean room suit worn by a person in the clean room lock. Correspondingly, a clean room lock adapter of a clean room suit may be a fastening element matching to another fastening element and allowing to couple the clean room suit to a clean room suit adapter of the clean room lock. Such a coupling may be a sealed coupling between an interior of the clean room suit and a fluid supply of the clean room lock. For instance, two correspondingly shaped and dimensioned fastening elements may be engaged to one another in a reversible or detachable manner to provide the adapter function.

The term "clean room suit" which may also be denoted as a bunny suit may denote an overall garment to be worn by a person in a clean room. One example is an all in one cover which may be appropriate for semiconductor and nanotechnology line production, technicians and process engineers as well as for people in similar rolls creating sterile products for the medical device and pharmaceutical industry. A clean room suit may cover the wearer to prevent skin and hair from being brought in contact with a clean room environment. A clean room suit may be integrally formed in one piece or may comprise several separate garment items worn tightly together.

The term "fabric" may particular denote any flexible material which can be used as a basis for a garment or a suit. For example, such a fabric may comprise a textile material which, in turn, may comprise a network of natural or artificial fibers such as thread or yarn. Thus, a fabric may be considered as an artifact made by weaving or felting or knitting or crocheting natural or synthetic fibers.

The term "person" may particularly denote a human being wishing to enter or leave a clean room. The term "person," should be considered as a human being of an average size, particularly between 1,50 m and 1,90 m in height and between 40 kg and 120 kg of weight. However, also children can be considered as persons in the context of the present application.

The term "fluid" may particularly denote any gas and/or liquid, wherein optionally also solid particles may be part of such a fluid. Gases of such fluids may particularly be basically inert gases such as oxygen molecules, nitrogen molecules, air or noble gases. Fluids may particularly include water and aqueous solutions and also liquid chemicals for instance for sterilization purposes.

According to an exemplary embodiment, an architecture for a clean room system is provided in which it may be dispensable that a person entering a clean room from an unclean side has to change clothing several times and has to pass multiple lock stages. This may be achieved by providing the person with a (for instance single) clean room suit to be worn at least over a majority portion of the body and having a clean room lock adapter allowing to couple a tube or the like of a clean room lock to the clean room lock adapter. The clean room lock adapter may guide the tube or the like towards an interior of the clean room suit so that for instance by the application of pressure a fluid may be pumped in an interior of the clean room suit so that both particles on an outer surface of the clean room suit as well as particles within a fabric layer of the clean room suit are promoted to move apart from the clean room suit, i.e. away from the person wearing the clean room suit, so that these particles may be removed from the person wearing the clean room suit and can be sucked off subsequently. By taking this measure, a very simple, save and efficient procedure of cleaning a person wearing a clean room suit is provided so that it may become possible that a person enters a clean room from an unclean side by a reduced number of clean room locks, for instance by only a few clean room locks or by only a single clean room lock.

In the following, further exemplary embodiments of the clean room suit will be explained. However, these embodiments also apply to the clean room lock, to the clean room arrangement and to the method.

The fabric may be configured to cover the entire body of the person, optionally with the exception of feet, hands and/or a part of the head of the person (particularly an eye portion may be free of the garment and may be covered by separate safety glasses). For instance, the clean room suit may be basically an overall suit, wherein only feet, hands and a part of the head (particularly including the eyes) may remain free of the fabric and may be protected by other components such as individual shoe pieces, glove pieces, protection glasses, etc.

The fabric may be configured to sealingly end at feet, hands and/or a part of the head when these body parts remain uncovered by the fabric. Therefore, at these end portions of the fabric where other clean room suit elements such as shoes, gloves or glasses are to be worn by the person, the fabric may seal the body within the fabric with regard to an environment, for hygienic reasons and to form a basically air-tight volume within the fabric which can hence be slightly inflated by pumping a pressurized gas in this volume via the clean room lock adapter. This may be achieved by an elastic band or the like which may be provided at end portions of the garment for instance close to the feet.

The fabric may comprise an inner fabric portion (such as a lining) to be placed directly on at least a part of the body (for instance, the inner fabric portion may be placed directly on the skin of the person, wherein it is possible that the person optionally wears underwear below the inner fabric portion). The fabric may also comprise an outer fabric portion (such as a shell fabric) to be exposed to an exterior environment of the clean room suit. In other words, the inner fabric portion may be arranged between the body and the outer fabric portion. Thus, the fabric may be made of two (or more) separate components. Such a construction may allow each of the components to be specifically adapted to the corresponding task of a respective component.

Particularly, the inner fabric portion and the outer fabric portion may be connected to one another (for instance in a sealed manner). For instance, these two fabric portions may be connected to one another by sewing, welding, gluing, etc. so that a single piece covering the majority of the body may be provided which eases the use for a user.

Furthermore, a fluidic lumen may be formed between the inner fabric portion and the outer fabric portion which can be in fluid communication with the clean room lock adapter so that a fluid such as a gas may be pumped in the lumen between the inner and the outer fabric portion. The material particularly of the outer fabric portion may be selected such that on the one hand the lumen within the outer fabric portion is sufficiently sealed to allow for some inflation of the clean room suit upon applying a pressurized gas flow to the lumen within the outer fabric portion. Simultaneously, the material particularly of the outer fabric portion may be selected such that on the other hand the outer fabric portion is sufficiently permeable for the pressurized gas to allow the gas to stream through the outer fabric portion, thereby carrying away particles within the outer fabric portion and at an outer surface of the outer fabric portion to clean the fabric.

According to another exemplary embodiment, the fabric of the clean room suit comprises one or more antimicrobial agents being capable of controlling microbial growth on clean room equipment, particularly on the clean room suit, wherein the one or more antimicrobial agents are of organic origin.

The term "clean room equipment", as used herein, refers to any goods that are required for a production process in a clean room environment including *inter alia* furniture, any types of devices or apparatuses (e.g., pipettes, robots, centrifuges, work benches, computer systems, and the like), laboratory supplies (e.g., tubes, culture dishes, pipette tips, pens, paper, notebooks, packaging containers, and the like), and particularly clean room clothing such as a clean room suit. The clean room clothing may further be provided with one or more antimicrobial agents of inorganic origin, for example gold and/or silver fibers incorporated in the fabrication materials.

The term "controlling microbial growth", as used herein, denotes any activity for completely inhibiting or at least reducing the growth of microorganisms such as bacteria, archaea, yeasts, fungi, and viruses. In particular, the term relates to biologically controlling growth of said microorganisms, that is, by employing biological means as "growth inhibitors or growth reducing agents."

The term "inhibiting", as used herein, is to be understood as not only to include the prevention of further growth but also the killing of microorganisms. The term "reducing", as used herein, denotes any decrease in microbial growth (i.e. the growth rate), for example, a decrease of at least 5%, 10%, 15%, 20%, 25%, 30%, 40%, 50%, 60%, 70%, 80%, 85%, 90% or 95% as compared to control conditions (i.e. in the absence of antimicrobial agents).

In specific exemplary embodiments, the antimicrobial agents are capable of controlling growth of extremo-tolerant microorganisms, that is, microorganisms specifically adapted to rather harsh environmental conditions (e.g., depletion of nutrition factors, environmental stresses, and the like) such as the conditions occurring in clean room environments. Numerous extremo-tolerant microorganisms are known in the art (see, for example, La Duc, M.T. et al. (2007) Appl. Environ. Microbiol. 73, 2600-2611; Moissl, C. et al (2007) FEMS Microbiol. Ecol. 61, 509-521).

The term "antimicrobial agent", as used herein, refers to any compound or means having an inhibitory (or antagonistic) effect on the growth of microorganisms, that is, agents that are capable of at least reducing the growth rate (e.g., bacteriostatic agents with respect to controlling the growth of bacteria) as well as agents that cause toxic effects (e.g., bactericide agents killing bacteria) provided that these compounds or means are of organic origin (i.e. composed of organic compounds).

The one or more antimicrobial agents may be arranged on any portion(s) of the fabric comprised in the clean room suit. Preferably, said agents are arranged on the outer portion of the fabric.

The arrangement of said antimicrobial agents may be accomplished by any physical and/or chemical contacting method known in the art. The contacting step may comprise a physical contacting method, particularly a method selected from the group consisting of spraying and gassing of the fabric with the one or more antimicrobial agents (present, e.g., in gaseous form, as an aerosol, a solution or a foam), for example by means of one or more spray heads, nozzles, pump-injectors, spray valves, and the like.

Additionally and/or alternatively, the contacting step may comprise a chemical coupling of the one or more antimicrobial agents (present, e.g., in gaseous form, as an aerosol, a solution or a foam) to the fabric. Chemical coupling can be achieved either by covalent or non-covalent bonding between the one or more antimicrobial agents and the fabric.

The term "covalent bonding" refers to a form of chemical bonding characterized by the sharing of one or more pairs of electrons between two components, producing a mutual attraction that holds the resultant chemical linkage (i.e. molecule) together. The term "non-covalent bonding" refers to a variety of interactions that are not covalent in nature, between molecules or parts of molecules that provide force to hold the molecules or parts of molecules together usually in a specific orientation or conformation. Such non-covalent interactions include *inter alia* ionic bonds, hydrophobic interactions, hydrogen bonds, Van-der-Waals forces, and dipole-dipole bonds.

The one or more antimicrobial agents may either be attached directly to the fabric or via a given linker molecule. Numerous linkers are known in the art. A skilled person is well aware of selecting a particular linker molecule appropriate for coupling a given antimicrobial agent to the fabric to be treated.

The one or more antimicrobial agents may be (chemically) coupled in a functionally active form or as an inactive precursor molecule that needs to be activated, e.g., by enzymatic cleavage, photolysis, UV radiation, and the like. In some exemplary embodiments, the antimicrobial agent(s) are activated simply be releasing it from the fabric on which they are aranged, for example via a cleavable linker molecule. In other exemplary embodiments, the one or more antimicrobial agents are incorporated or embedded in vesicles, layers, coatings or other structures arranged on or incorporated in the fabric (e.g., located on the outer portion of the fabric or between the inner and outer portions of the fabric) from that they are released, for example, in form of a sustained release formulation over a given period of time or upon a particular trigger such as heat, radiation or the presence of microbial contaminants (i.e. the agents are incorporated in a bioactive layer). All such configurations are well established in the art.

Preferably, the one or more antimicrobial agents are selected from the group consisting of microorganisms, preferably of bacteria, yeasts, and fungi, and metabolites produced by said microorganisms.

"Antagonistic" microorganisms that may be employed herein are characterized by one or more structural and/or functional properties conferring to them a selection advantage as compared to the microorganisms to be treated, thus resulting in a superior growth rate. Such organisms include *inter alia* (i) microorganisms that compete with the microorganisms to be treated for any nutrients and/or any trace elements (e.g., by depleting Fe³⁺ via the secretion of iron-chelating siderophores) and/or for living space, (ii) microorganisms that produce (and optionally secrete in form of exoenzymes) lytic compounds such as glucanases, chitinases, cellulases, hydrolases (e.g. lysozyme), and lyases, and (iii) microorganisms that produce antimicrobials/antibiotics, toxins, volatile organic compounds (VOCs; i.e. chemical compounds that have high enough vapor pressures under normal conditions to significantly vaporize and to enter the atmosphere) and/or biosurfactants (i.e. surface-active compounds that enhance the emulsification of hydrocarbons, have the potential to solubilize hydrocarbons and increase their availability for microbial degradation).

Examples of antibiotics include *inter alia* β-lactam antibiotics (e.g., penicillin, cephalosporin), tetracyclins, aminoglycoside (e.g., streptomycin, kanamycin, gentamicin), and macrolides (e.g., erythromycin). The term "toxins", as used herein, also includes bacteriocins, that is, proteinaceous toxins produced by bacteria to inhibit the growth of similar or closely related bacterial species. Examples of such toxins are, e.g., colicin, halocin, nisin, sakacin, and vibriocin. Examples of volatile organic compounds include *inter alia* methane and formaldehyde. Finally, examples of biosurfactants include *inter alia* mycolic acid, glycolipids, polysaccharide-lipid complexes, lipoproteins or lipopeptides, and phospholipids.

Instead of and/or in addition to the microorganisms as such one or more metabolites produced by said microorganisms, preferably the exoenzymes, antibiotics, volatile organic compounds, and biosurfactants mentioned above, may be employed herein. The term "produced by said microorganisms", as used herein, is not to be understood that practicing it is necessarily required to isolate any "growth inhibitory" metabolites from said microorganisms. Rather, the term merely refers to the fact that said metabolites represent "antagonistic factors" of natural origin produced (and optionally secreted) by microorganisms.

Accordingly, the "antagonistic" metabolites employed herein may not only be naturally occurring metabolites (i.e. metabolites isolated from antagonistic microorganisms as defined herein) but also corresponding metabolites that are chemically synthesized or generated by means of recombinant gene technology (see, for example, Sambrook, J. et al. (1989) Molecular, Cloning: A Laboratory Manual, 2nd ed., Cold Spring Harbor Laboratory Press, Cold Spring Harbor, NY).

In some other exemplary embodiments, one or more of the microorganisms and/or metabolites are isolated or enriched from plants, preferably from the rhizosphere, endosphere or phyllosphere of plants. The term "rhizosphere", as used herein, denotes the region (layer) of soil (including the root surfaces) that is directly influenced by secretions of a plant's roots and root-associated soil microorganisms. The term "endosphere", as used herein, denotes the interior of a plant. Finally,, the term "phyllosphere", as used herein, refers to a plant's leaf surfaces or the total above-ground surfaces of a plant.

Examples of "antagonistic" microorganisms that can be isolated from the rhizosphere include *inter alia Chryseobacterium spec., Enterobacter spec., Salmonella typhimurium, Serratia spec., Stenotrophomonas maltophilia, Staphylococcus spec.,* and *Pseudomonas aeruginosa.* Examples of "antagonistic" microorganisms that can be isolated from the endosphere include *inter alia Staphylococcus epidermidis, Pseudomonas aeruginosa, Streptococcus mitis, Serratia* spec., *Enterobacter spec.,* and *Stenotrophomonas maltophilia.* Finally, examples of "antagonistic" microorganisms that can be isolated from the phyllosphere include *inter alia Serratia plymuthica, Pseudomonas putida,* and *Pantoea agglomerans.*

The one or more antimicrobial agents employed in a given setting may all be isolated or enriched from a single plant species (variety) or they may be derived from at least two different plant species of the same plant genus or they may be isolated or enriched from at least two plants belonging to different genera.

In further particular exemplary embodiments, the plants are selected from the dicotyledon plants (also referred to as "dicotyledons" or "dicots"). The term "dicotyledon plants", as used herein, denotes the group of flowering plants (i.e. angiosperms) whose seed typically has two embryonic leaves or cotyledons. However, in other embodiments monocotyledon plants (also referred to as "monocotyledons" or "monocots") having only a single cotyledon may be employed as well.

Examples of dicotyledons to be used herein include *inter alia Fragaria spec.* (i.e. strawberry), *Brassica napus* (i.e. rape or oilseed rape), *Cucurbita pepo* (i.e. pumpkin or squash), *Beta vulgaris* (i.e. beet), *Solanum tuberosum* (i.e. potato), *Sphagnum spec.* (i.e. peat moss), *Viscum album* (i.e. mistletoe), and *Acer tataricum* (i.e. Tatar maple).

The term "isolated", as used herein, denotes that the antimicrobial agents used herein are present in pure (or almost pure) form, for example at least 75%, 80%, 85%, 90%, 95% 98% or 99% pure or completely pure (i.e. 100% pure). The term "enriched", as used herein, denotes a crude preparation (e.g., a plant extract) subjected to one or more basic purification steps (e.g., a filtration, a precipitation or a centrifugation step), thus resulting in the accumulation of the antimicrobial agents in such preparation which, however, does still include significant impurities. For example, such enriched preparations may be at least 20%, 30%, 40%, 50%, 60% or 70% pure.

The one or more antimicrobial agents employed herein may comprise microorganisms of any one or more microbial species exhibiting an antagonistic (i.e. growth inhibiting or at least growth reducing) behavior towards one or more microorganisms inhabiting a clean room setting. Examples of such "antagonistic" microorganisms include *inter alia* any species belonging to the group consisting of the genera *Acinetobacter, Aeromonas, Agrobacterium, Amycolatopsis, Arthrobacter, Bacillus, Brevundimonas, Burkholderia, Cellulomonas, Citrobacter, Clavibacter, Chromobacterium, Comamonas, Corynebacterium, Chryseomonas, Curtobacterium, Delftia, Enterobacter, Enterococcus, Erwinia, Escherichia, Flavobacterium, Frigoribacterium, Gordonia, Herbaspirillum, Klebsiella, Kitasatospora, Kocuria, Kytococcus, Lactobacillus, Leifsonia, Lysobacter, Methylobacterium, Microbacterium, Micrococcus, Micromonospora, Morganella, Neisseria, Nocardia, Oerskovia, Paenibacillus, Pantoea, Pectobacterium, Proteus, Rahnella, Ralstonia, Rhizomonas, Salmonella, Serratia, Sphingobacterium, Sphingomonas, Staphylococcus, Streptomyces, Stenotrophomonas, Streptoverticillium, Tsukamurella, Variovorax, Xanthobacter,* and *Xanthomonas.* All these genera are well known in the art (cf., for example, Garrity, G.M. (ed.) Bergey's Manual of Systematic Bacteriology, Vol. II - The Proteobacteria (Brenner, D.J. et al., eds.), 2nd ed. (2005), Springer Press, New York).

The antimicrobial agents comprised in the fabric of the clean room suit may include any species of antagonistic microorganisms or any combination of any one or more species belonging to any one or more genera of the antagonistic microorganisms referred to herein above. For example, two or more species of microorganisms belonging to the same genus (e.g., *Bacillus, Microbacterium* or *Stenotrophomonas)* may be employed as well as two or more species of microorganisms belonging to at least two different genera (e.g., (*Enterobacter* and *Paenibacillus*) or (*Cellulomonas* and *Neisseria* and *Xanthobacter*))*.*

Preferably, the one or more antimicrobial agents comprise microorganisms of the species *Serratia plymuthica* and/or of the species *Pantoea agglomerans,* and/or the one or more antimicrobial agents comprise one or more metabolites selected from the group consisting of exoenzymes, antibiotics, volatile organic compounds, and biosurfactants.

The term "one or more" (antimicrobial agents), as used herein, denotes that a single type of microbial agent may be employed (that is, e.g., a particular microorganism (i.e. a particular species) or a specific metabolite) or at least two different types of microbial agents, wherein the at least two different types may be, e.g., at least two different species of microorganisms (that may or may not belong to the same genus) or at least two different types of metabolites (that may or may not belong to the same class of chemical compounds) or at least one microorganism and at least one metabolite. Accordingly, the one or more antimicrobial agents employed herein may comprise, for example, three different species of microorganisms (e.g., *Serratia plymuthica, Pantoea agglomerans,* and *Stenotrophomonas maltophilia*)*,* or four different types of metabolites (e.g., a glucanase, kanamycin, and two biosurfactants), or a combination of two different species of microorganisms (e.g., *Serratia plymuthica,* and *Pantoea agglomerans)* with two different types of metabolites (e.g. two biosurfactants).

The choice of (a) particular antimicrobial agent(s) for a given scenario may depend *inter alia* on the extent of microbial growth (i.e. the quantity) present on or supposed to be present on the clean room equipment as well as on the nature (i.e. the quality) of the contaminants present. The skilled person is also well aware how to select one or more antimicrobial agents for a given scenario.

The clean room lock adapter may be arranged on and/or in the outer fabric portion. In contrast to this, the inner fabric portion may be free of the adapter. Therefore, it may be easily possible even for an unskilled user to connect a clean room suit adapter to the clean room lock adapter for providing fluid communication between clean room suit and clean room lock (particularly a tube thereof) in an airtight manner.

In an embodiment, the inner fabric portion may be made of a first material and the outer fabric portion may be made of a second material differing from the first materials. Hence, the material selection of the inner fabric portion and of the outer fabric portion may be different and may be specifically adjusted with regard to corresponding tasks of these respective fabric portions. A task of the inner fabric portion is to prevent particles from the human body to reach the outer surrounding of the fabric, i.e. a cabin of the clean room lock. The outer fabric portion may have the task to prevent any particles from adhering to this fabric portion, in order to keep the clean room clean.

Accordingly, the inner fabric portion may be made of a breathable, eudermic material being impermeable for particles from the body of the person. It may happen that on a surface of the body of the person, small particles are present which should not be permeated through the inner fabric portion towards an outer portion of the clean room suit. A breathable and eudermic material not only allows for a convenient wear of the clean room suit by a person, but also prevents or suppresses sweating or the like which can also be the origin of impurities.

The outer fabric portion may be made of a material being repellent against particles such as dust and/or germs and being clean room compatible. Therefore, such an outer fabric portion may be made of a material which is not prone to particle adhesion to an outer portion thereof or to pores within the fabric portion. Therefore, by a corresponding selection of such a material, the performance of the clean room suit may be further improved.

It should be mentioned that the separation of the fabric into two or more separate fabric portions may be advantageous, but that in other embodiments, the fabric may be made of a single component, piece or material.

The clean room lock adapter of the clean room suit may be arranged on and/or in the fabric at a position of an abdomen of the person wearing the clean room suit. For example, the clean room lock adapter may be approximately positioned close to a navel of the person. Such a position is an appropriate position enabling even an unskilled user to easily connect a hose or tube or the like of the clean room lock to the adapter of the clean room suit for providing a pressurized gas beam for cleaning purposes within the clean room suit.

The clean room lock adapter may comprise a connection flange configured for mounting on a correspondingly configured clean room lock. Therefore, the clean room lock adapter and the clean room suit adapter may be correspondingly shaped sized and dimensioned so as to allow for a mating connection between these two adapter portions, in a reversible or detachable way. Preferably, such a connection may result in a gas-tight configuration so that it may be possible to provide a fluid such as a gas via the connected clean room lock adapter/clean room suit adapter without leakage. The assembly of clean room lock adapter and clean room suit adapter to one another should also be reversible so that, for instance with a single hand motion of the user, the user may couple or decouple clean room suit and clean room lock to one another or decouple them from one another.

Particularly, the connection flange may be configured for mounting on the correspondingly configured clean room lock by a snap-in connection, a bayonet connection, a screwing connection, or any other fluid sealed-connection. With such easily operable connection systems, it may be ensured that the two adapters may selectively engage one another or disengage one another by a single hand motion which allows to operate the system in a short time and without the need of experienced skills of a user.

The clean room lock adapter may comprise a membrane adapted for being movable upon applying a pressure on the membrane. Such a membrane may be made of one or more diaphragms which can provide some kind of sealing function for sealing an interior of the clean room suit in the absence of an external pressure, i.e. below a predefined threshold value. When this threshold value is exceeded, the membrane may be deformed so as to allow a fluid transfer from a hose and both adapters through the membrane, therefore allowing to pump up an interior of the clean room suit by applying a pressure via the clean room lock adapter and the membrane.

According to an exemplary embodiment, it is possible that an electrical pickup member (for instance a contactor or an activation switch or any kind of sensor) may be arranged on or at the membrane or at another portion of the clean room lock adapter. Such an electrical pickup member may be capable of sensing, for instance, whether the clean room lock adapter is presently connected to a clean room suit adapter. A corresponding sensor signal may be a necessary condition before execution of a cleaning procedure is approved by an automatic control unit such as a microprocessor. It is also possible that such an electrical pickup member provides a measurement signal indicative of the presence or absence of an external pressure applied via the adapter to an interior of the clean room suit.

The clean room lock adapter may be configured for conveying or blowing a fluid provided by the clean room lock to an interior of the fabric and from there through the fabric so that particles on and/or in the fabric are moved out of the fabric towards the exterior of the clean room suit. In other words, it may be possible to connect a gas hose to the clean room lock adapter allowing to provide a gas pressure which may result, due to some kind of sealing connection of the clean room lock adapter and the clean room suit adapter, in an overpressure in an interior of the clean room suit. Such an overpressure may generate a force acting on potentially present particles on an exterior wall of the clean room suit and/or within the fabric. Thus, such particles may be blown out of the clean room suit towards a portion of the clean room lock surrounding the clean room suit. Such particles can then be sucked off so that an efficient cleaning of the clean room suit can take place in a very short time.

It should be mentioned that, as an alternative to an overpressure, it is also possible that other forces promoting removal of particles from an interior towards an exterior of the clean room suit can be applied, for instance the removal of such particles by increasing the temperature within the clean room suit, by generating concentration differences between an interior and an exterior of the fabric for promoting equilibration of the concentration gradient by particle migration or any other suitable measure such as the use of electric and/or magnetic forces.

In an embodiment, the clean room suit may comprise goggles configured to cover at least the eyes of the person. Such a pair of protection goggles may have a plurality of synergetic effects. Firstly, the goggles may prevent dust on a face of a person to be exposed to an environment. Secondly, such goggles may also serve as a protection against further optional cleaning procedures such as the introduction of a sterilizing chemical agent in the clean room lock and/or the introduction of sterilizing ultraviolet radiation (or electromagnetic radiation of any other appropriate wavelength range) into a cabin of the clean room lock. Therefore, a corresponding chemical inertness of the material of the goggles as well as a radiation filter function for filtering potentially harmful frequencies of such ultraviolet radiation may be considered in the goggles.

Furthermore, the clean room suit may additionally comprise shoes configured to cover feet of the person. The clean room suit may also comprise gloves configured to cover hands of the person. Therefore, basically the entire body of the person may be covered with a protection material.

Next, further exemplary embodiments of the clean room lock will be explained. However, these embodiments also apply to the clean room suit, to the clean room arrangement and to the method.

The clean room suit adapter of the clean room lock may comprise a connection flange configured for mounting on a correspondingly configured clean room suit, particularly a correspondingly configured clean room lock adapter of the clean room suit. In other words, the adapters of clean room lock and clean room suit may be configured to correspond to one another.

Such a connection flange may be configured for mounting on the correspondingly configured clean room suit by a snap-in connection, a bayonet connection, a screwing connection, or any other fluid sealed-connection.

The clean room lock may comprise a tube connected to the clean room suit adapter and configured for supplying a pressurized fluid to the clean room suit adapter. Such a tube may be anchored in a wall, for instance a sidewall, enclosing a cabin of the clean room lock. Thus, a vertical wall accommodating a fluid supply unit may be adapted for supplying a fluid to the tube. Such a fluid supply unit may for instance be a gas container for providing an overpressure or may also be a gas supply system of a hospital, a plant or the like.

Thus, the connection may be such that a fluid supplied via a hose of the clean room lock (which hose may be mounted on or anchored at a wall or the like of the clean room lock and may be in fluid communication with a gas supply) is conducted through the clean room suit adapter and the clean room lock adapter to an interior of the clean room suit.

The clean room suit adapter may be configured for providing a pressurized fluid to the clean room suit so that the particles on and/or in the clean room suit are moved out of the clean room suit towards the exterior of the clean room suit. For instance, the pressure provided via a fluid conduit or tube towards the clean room suit adapter may be supplied with an overpressure of more than one atmosphere, for instance in a range between 1.2 bar and 2 bar, so as to pump up an interior of the clean room suit to efficiently move particles from the clean room suit towards a surrounding thereof under the influence of an overpressure force.

In an embodiment, the clean room lock may comprise a cabin being spatially delimited by (for instance four) side walls, a bottom wall and a ceiling, wherein the cabin may be configured so that a standing person properly fits into the cabin. For instance, a ground area of the cabin may be in a range between 1 m² and 4 m², for instance may be about 2.5 m². Within such a cabin, it should be possible that the person can stand without suffering from claustrophobic feelings. At the same time, the volume of the cabin should be sufficiently small so that a cleaning treatment has to be applied only to a small volume, thereby keeping the system simple and cheap in operation.

The bottom wall of the clean room lock may comprise a marker indicative of a target position of feet of the person standing in the cabin. For example, such a marker may comprise any optical (for instance boundary lines delimiting foot portions in a manner which is intuitive for a person) and/or haptic (for instance recesses in the floor delimiting foot portions in a manner which is intuitive for a person) indicia indicating at which positions a person should put her or his foot or feet on. In view of a high efficiency of a specific cleaning procedure to be executed, it may be advantageous that the person is located at the correct position. It is also possible that a circle (or any other area) is indicated on the ground within which circle the person should stand. A correct standing positions of the person may be controlled via one or more appropriate sensors.

For example, the marker may be arranged so that the target position corresponds to a standing position in which the person stands in the cabin with (for example slightly) straddled legs. In such a configuration, the surface of the clean room suit between the legs of the person may be also exposed to a cleaning procedure which may therefore be applied to this surface as well, thereby preventing undesired shadowing effects'and ensuring a complete cleaning.

The bottom wall may comprise a transparent material. The material of the bottom wall may be transparent for electromagnetic radiation (such as UV) which can be applied from a bottom of the clean room lock. For example, such an irradiation of the bottom may also allow to cleaning a shoe sole of the clean room suit. Particularly, the material of the bottom wall may be transparent for ultraviolet radiation which may be used for a decontamination or sterilization through the bottom wall.

The clean room lock may further comprise one or more ultraviolet radiation sources. Such ultraviolet radiation sources may be accommodated below the bottom and may be configured for irradiating the clean room suit from below, i.e. vertically, with ultraviolet radiation. Additionally or alternatively, such ultraviolet radiation sources may be accommodated on or in side walls of a cabin of the lock room and may be configured for irradiating the clean room suit laterally or horizontally with ultraviolet radiation. Additionally or alternatively, such ultraviolet radiation sources may be accommodated on or in a ceiling wall of a cabin of the lock room and may be configured for irradiating the clean room suit vertically from above with ultraviolet radiation. For instance, UV-C may be a suitable wavelength range for ultraviolet radiation usable for a decontamination of a surface portion of the clean room suit. Particularly, shoe soles of the clean room suit and a portion between the legs of the person can be decontaminated by taking this measure.

A first one of the (for instance four) surrounding side walls (wherein a circular side wall is possible as well) may comprise a first door connecting the clean room lock with an exterior of the clean room, i.e. an unclean side. Such a door may be gas-tight when closed and may be opened to allow a person to leave the clean room lock. Furthermore, a second one of the side walls may comprise a second door connecting the clean room lock with an interior of the clean room, i.e. a clean side. The two side walls accommodating the two doors may be arranged to oppose one another. Both doors may be gas-tight when closed. It is possible that the doors include a transparent window such as a glass element so that the person in the clean room lock may look out of the doors, thereby preventing any tightness feelings.

The first door and the second door may be lockable, particularly may be mutually lockable. A corresponding locking mechanism may be a purely mechanical locking mechanism or may be an electronic mechanism which automatically locks and unlocks the corresponding doors in order to prevent undesired misuse of the doors which could deteriorate the cleaning conditions in the clean room lock or even in the clean room. However, it may be possible to provide an emergency opening button allowing a user in the case of an emergency to open the doors immediately.

The clean room lock may comprise a gas jet unit, particularly an air jet unit, adapted for blowing a gas to the cabin around an exterior of the clean room suit. Such a feature may be particularly advantageous in combination with the application of an overpressure on an interior of the clean room suit. The latter measure may generate a stream of particles around the clean room suit which particles have been removed from the clean room suit due to an applied overpressure. By providing an air jet around the clean room suit (for instance a gas flow direction of the air jet can be defined using the assumption that the person stands on markers indicating a target standing position), such freed particles may be removed from the surrounding of the clean room suit, thereby promoting a proper cleaning of the clean room suit and preventing such particles from a re-deposition on the clean room suit.

For instance, it is possible that such a gas jet unit comprises a plurality of blowing nozzles, each capable of blowing the gas around an exterior of the clean room suit. For instance, the plurality of nozzles may be arranged in a plurality of groups, wherein nozzles of the same group may be arranged along a vertical row and each blow along a same direction, and nozzles of different groups are arranged to blow along different directions. Therefore, it may be possible that nozzles of a group generate a planar stream removing particles along a respective plane adjacent the clean room suit. When a plurality of such gas jet planes intersect one another, an efficient discharge of particles removed from the clean room suit may be achieved. For instance, it is possible that four such groups are arranged providing gas jet planes with an intersection angle of about 90°.

It is possible that the plurality of nozzles are arranged along the sidewalls of the cabin. Thus, the sidewalls may be used as carriers for gas tubes connected to these nozzles, and the nozzles may be assembled to such a sidewall or to a corner between sidewalls.

The clean room lock may further comprise a decontamination unit (such as a chemical decontamination unit) adapted for ejecting a decontamination agent (such as a chemical decontamination agent) for decontaminating the clean room suit. Thus, in addition to the removal of particles from the clean room suit, a protection against biological germs may be further increased by for instance chemically decontaminating a surface of the clean room suit, which may be advantageously for instance for medical and pharmaceutical applications.

Such a decontamination unit may particularly supply the decontamination agent to the cabin via the ceiling. However, it is also possible that other sidewalls or also the bottom wall of the cabin provide nozzles for ejecting a decontamination agent such as diethyl-hexyl-sebacate (DEHS) or hydrogen peroxide (H₂O₂). Therefore, the sterilization efficiency may be increased. Although the person may be properly protected against used chemicals by the clean room suit, it might be advantageous to use a decontamination agent which is not harmful for human beings.

Moreover, a suction unit may be provided for sucking gas off or from the clean room lock. Such a suction unit may locally generate a negative pressure, for instance to remove a chemical decontamination agent after interaction with the clean room suit, particles, etc. In view of the gravity force tending to accumulate matter close to the bottom, it may be advantageous that the suction unit is arranged at or close to a bottom position of the sidewalls, so that the ejection direction of the chemical decontamination agent in combination with the gravitational force result in a removal of a large portion of the injected chemical decontamination agent by the suction unit.

One or more filter units may be arranged in a suction line or in fluid communication with the suction unit so that the fluid sucked by the suction unit is filtered by the filter unit. The suction unit may, additionally or alternatively to the removal of the chemical decontamination agent, also suck gas including particles which have previously been removed from the clean room suit. These particles may be captured in a filter of such a filter unit at the entrance of the suction unit. Particularly, a pre-filter may be arranged at such a position. If desired, such a pre-filter may be combined with an additional filter for suspended particles.

The clean room lock may further comprise a gas supply unit adapted for re-supplying filtered gas to the clean room lock after filtering. By taking this measure, a circulating gas system may be provided which allows to recycle the gas sucked from the clean room lock to be injected into the clean room lock again after purification or removal of the particles.

In an embodiment, the clean room lock may comprise a handle (more particularly two handles, one for each hand of the human being), particularly a bow-type handle. Such a handle may be arranged above the person so that hands of the person, when raised above a head, can grip the handle. By taking this measure, undesired shadowing effects, for instance of portions below axles of the human, may be prevented and a large surface of the clean room suit may be exposed to the cleaning procedure.

The handle may comprise one or more sensors which may be configured for generating a signal triggering start of a predefined cleaning procedure upon detecting that the hands of the person grip the handle. It may be advantageous that the proper gripping of the handle by the person is detected before the cleaning procedure starts. This prevents an improper cleaning procedure in which for instance surface portions below the axle of the person are not exposed to the cleaning procedure.

The clean room lock may comprise a control unit adapted for centrally controlling operation of the cleaning procedure. Such a control unit may have processing and storage capability and may centrally control operation of the entire system. More particularly, the control unit may be adapted for automatically executing a predefined cleaning procedure. It may be possible that a storage unit stores a plurality of predefined cleaning procedures, each appropriate for a specific application, so that a corresponding one of these cleaning procedures may be selected for a specific application.

In an embodiment, the control unit may be configured for automatically executing the predefined cleaning procedure only when simultaneously at least the following three conditions are fulfilled:
1. It should be ensured that the clean room suit adapter is coupled to the clean room lock adapter. Only in this case, the cleaning by blowing out particles from the surface and from an interior of the fabric of the clean room suit may be ensured. For this purpose, a corresponding sensor may be arranged at one of the adapters to provide a specific trigger signal when a corresponding coupling between the two adapter portions is detected;
2. Feet of the person have to stand at a target position; only when this condition is fulfilled, a proper cleaning of the shoe soles may be ensured and also shadowing effects between the legs of the person may be prevented;
3. Hands of the person have to grip the handle; only when this is detected, for instance using one or more sensors, it can be ensured that the axle portions are prevented from being shadowed during a cleaning procedure.

Next, further exemplary embodiment of the clean room arrangement will be explained. However, these embodiments also apply to the clean room suit, to the clean room lock and to the method.

The clean room arrangement may additionally comprise a clean room. The clean room lock may be spatially arranged between an interior and an exterior of the clean room. Therefore, when a person comes from an unclean surrounding, this person first has to pass the clean room lock via one door thereof, before, after having passed the cleaning procedure within the clean room lock successfully, the person may open an opposed door which allows access from the clean room lock towards the clean room. It is possible that the clean room arrangement is used in either direction, i.e. either from the unclean side to a clean side or from the clean side to the unclean side.

According to an exemplary embodiment, it is possible that one or multiple of the described clean room locks is or are provided between the unclean environment and the clean room. The selection of the number (one or more) of clean room locks between clean room and unclean environment may depend on purity requirements of a specific application.

For example, the clean room arrangement may be used in the context of microtechnology clean rooms, semiconductor technology clean rooms, pharmaceutical clean rooms, chemical clean rooms, a stock breeding clean room, a medical clean room, a pharmacy clean room, or a radioactivity clean room (for instance in a nuclear power plant). Other applications are of course possible as well.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.
Fig. 1 illustrates a perspective view of a clean room lock according to an exemplary embodiment of the invention in which a person wearing a clean room suit according to an exemplary embodiment is presently located.
Fig. 2 illustrates a cross-sectional view of a clean room suit according to an exemplary embodiment of the invention.
Fig. 3 and Fig. 4 show two partially exploded views of a clean room suit from a bottom position according to an exemplary embodiment, wherein Fig. 3 relates to a view from a clean side and Fig. 4 relates to a view from an unclean side.
Fig. 5 and Fig. 6 show side views of access doors of a clean room lock according to an exemplary embodiment, wherein Fig. 5 relates to a view from a clean side and Fig. 6 relates to a view from an unclean side.
Fig. 7 illustrates a top view of the clean room lock of Fig. 5 and Fig. 6.
Fig. 8 shows a flowchart of a method according to an exemplary embodiment of the invention.

The illustration in the drawing is schematically. In different drawings, similar or identical elements are provided with the same reference signs.

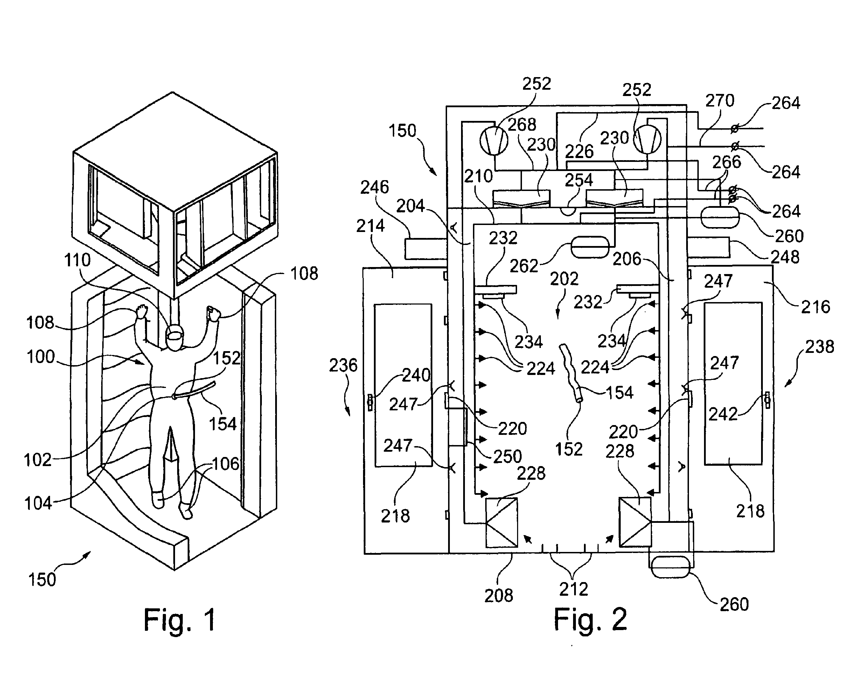

**Fig. 1** illustrates a person wearing a clean room suit 100 according to an exemplary embodiment of the invention and standing within a clean room lock 150 according to an exemplary embodiment of the invention.

The clean room suit 100 comprises an overall-like fabric 102 covering a major portion of a body of the person, and therefore being configured as an overall clean room suit. Close to a navel of the person, a clean room lock adapter 104 is integrated in the fabric 102. In Fig. 1, clean room lock adapter 104 is detachably coupled to a clean room suit adapter 152 of the clean room lock 150. Clean room suit adapter 152 is, in turn, coupled to a gas-tight tube 154. When gas is supplied from a gas supply container (integrated in a side wall and hence not shown) via the tube 154 and the adapters 152, 104 providing a sealed connection, the fluid is supplied to an interior of the fabric 102 which can be therefore slightly inflated. Consequently, particles which are potentially located on an outer surface of the fabric 102 and/or in pores within the fabric 102 will be blown out of the fabric 102 towards a surrounding volume of the clean room lock 150.

As can be taken from Fig. 1, only the hands, the feet and an eye portion of the person are not covered by the fabric 102. However, the feet are covered with clean room shoes 106 being sealedly connected with the fabric 102. Furthermore, the hands of the person are covered by gloves 108 being sealedly connected with the fabric 102 and the eyes are covered with protection goggles 110. The goggles 110 may comprise a wavelength selective filter to provide protection against UV radiation.

Although not shown in Fig. 1, the clean room suit 102 is made of an inner fabric directly attached on the skin of the person and an outer fabric exposed to the clean room lock 150. The inner fabric portion and the outer fabric portion are connected to one another by sewing, wherein an airtight lumen is formed between the outer and the inner fabric portion which can be filled with gas via the tube 154 and the adapters 152, 104. The inner fabric portion is breathable, eudermic and impermeable for particles originating from the body of the person, whereas the outer fabric portion is repellent against particles and clean room compatible.

Although not shown in Fig. 1, a membrane and an electrical pickup are provided at the clean room lock adapter 104 so that an interior of the clean room suit 100 is only in fluid communication with environment when a pressure is supplied deforming the membrane. Furthermore, the electrical pickup can detect the connection of the adapters 152, 104 to one another and may generate a corresponding sensor signal transmittable to a control unit 246 (see Fig. 2).

Details regarding the clean room lock 150 will be described in more detail referring to **Fig. 2****.**

The clean room lock 150 encloses a person cabin 202 having sidewalls 204, 206, a bottom wall 208 and a ceiling wall 210. The dimension of the cabin 204 is so that a standing adult person fits properly into the cabin 202. Markers 212 (for instance a target circle or individual foot markers) are provided on the transparent bottom wall 210 to indicate a position where the person should stand in the cabin 202. When the feet of the person are within the marker portions 212, the person stands in the cabin 202 with slightly straggled legs, for instance with an angle of 15° between the legs.

The material of the bottom wall 208 is transparent for UV radiation, for instance may be a quartz glass plate with an aluminium reflector. Although not shown in Fig. 2, an ultraviolet radiation source may be arranged below the transparent bottom 208 so as to irradiate a surface of the clean room suit 100, particularly a sole of the clean room shoes 106, with ultraviolet radiation for sterilization purposes. Also close to the sidewalls 204, 206, a number of UV sources 247 are arranged for laterally irradiating the cabin 202 with UV radiation for sterilization purposes.

Sidewall 204 comprises a first door 214, whereas sidewall 206 comprises a second door 216. The doors 214, 216 each have a glass element 218. The doors 214, 216 also have a corresponding electrical closure mechanism 220. Grips of the doors 214, 216 are indicated with reference numerals 240 and 242, respectively.

A gas jet system is provided which comprises nozzle rows each having eight nozzles 224 for blowing a gas towards the cabin 202, thereby also directing a gas flow around an exterior of the clean room suit 100. Via conduits 266, a chemical decontamination medium such as DEHS (diethyl-hexyl-sebacate) may be supplied in the cabin 202. The chemical decontamination agent may be supplied to the cabin 202 via the ceiling 210 and/or via the side walls 204, 206.

A suction system may provide an under pressure close to the bottom wall 208 of the cabin 202 and may suck off gas and/or liquid from the cabin 202 via pre-filters 228 and filters for suspended particles 230. Ventilators 252 may provide a corresponding pump performance. After filtering and hence cleaning, the filtered gas can be supplied back to the cabin 202.

Bow-type handles 232 are arranged in the cabin 202 above the person so that arms of the person are raised above a head of a person when the hands of the person grip the handles 232. The handles 232 comprise touch sensors 234, wherein the touch sensors 234 generate a signal triggering start of a predefined cleaning procedure upon detecting that the hands of the persons properly grip the handle 232, and therefore also touch the touch sensors 234. Other kinds of sensors are of course possible.

A clean side at which a clean room is directly connected is indicated with reference numeral 236, whereas an unclean side (a surrounding of the clean room) is indicated with reference numeral 238.

A user interface 246 at the clean side 236 allows a user to control an operation mode of the cleaning procedure, allows for a control of the ultraviolet decontamination and may house a central control unit for controlling the system 100, 150. A further user interface 248 is provided as well at the unclean side 238. A display panel 250 which, inter alia, includes a start button and an emergency stop and exit button is shown in the cabin 202 as well.

A motion sensor 254 is arranged at the ceiling wall 210 and may detect when the person moves after starting of a cleaning performance in the cabin 202 and can take corresponding measures, for instance may terminate the cleaning procedure or may repeat the cleaning procedure.

PDAH units 260 can be seen at various positions. A PDAL unit 262 is shown as well.

Control valves 264 can be controlled individually by the control unit 246 or by a human operator, for instance for activating or deactivating a DEHS injection (which may be measured as well) via fluid conduits 266.

Air delivery can be achieved via a conduit 226, whereas exhaust air can be conducted via a conduit 270.

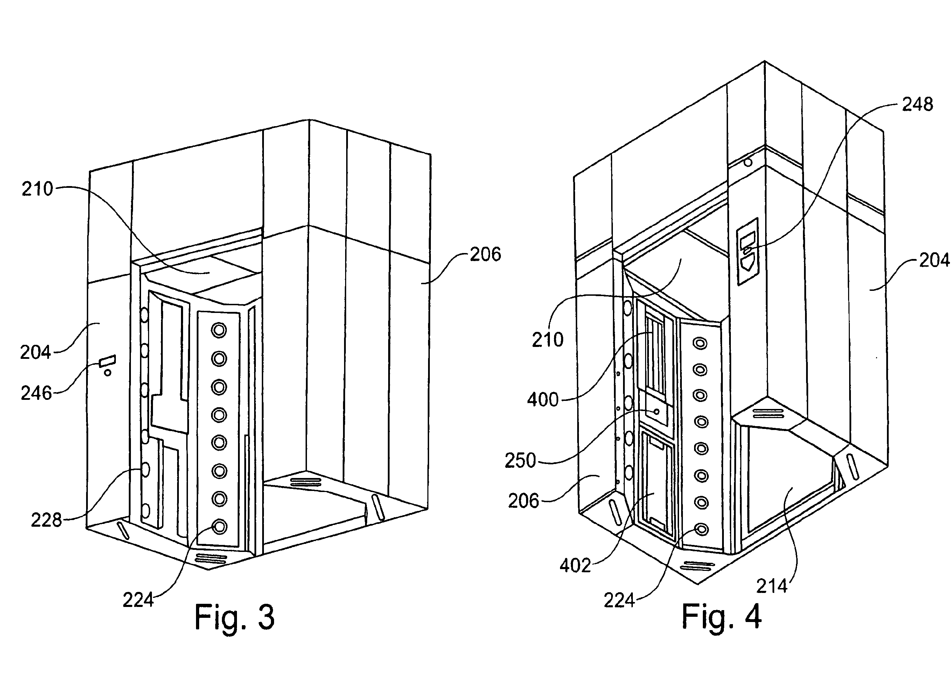

**Fig. 3** shows a partially exploded bottom view of the clean room lock 150 from the clean side 236 without showing the door 214.

In contrast to this, **Fig. 4** shows a partially exploded bottom view of the clean room lock 150 from the unclean side 238 without showing the door 216. An illumination 400 is shown. Furthermore, an UVC unit 402 is shown in Fig. 4 as well.

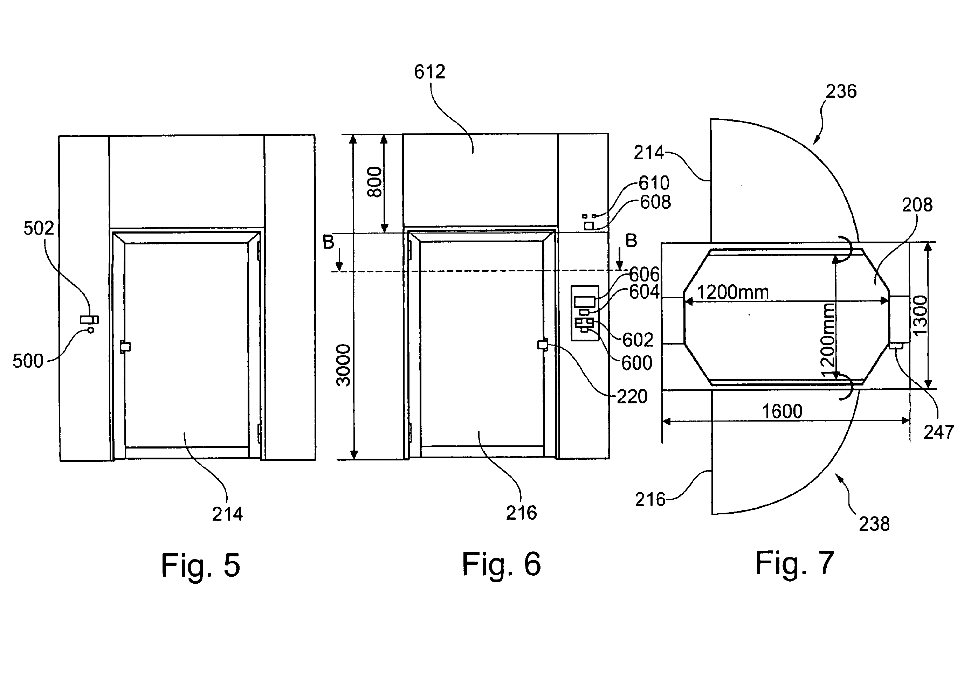

**Fig. 5** shows a plan view on the clean side 236 showing an emergency off switch 500 and a signal illumination panel 502.

**Fig. 6** shows a plan view on the unclean side 238. An emergency off button 600, a signal illumination panel 602, an operation panel for operating an UVC unit 604 and an additional operation panel 606 are shown. A main switch 608 and measurement ports 610 are shown as well. Ventilators and filters are integrated in the air unit 612.

**Fig. 7** shows a plan view on the ceiling 210 thereby also showing the clean side 236 and the unclean side 238. The UVC units 247 are indicated as well.

In the following, some basic recognitions of the present inventor will be explained based on which exemplary embodiments of the invention have been developed.

Clean rooms can be distinguished regarding design, technology and use, as well as operation and regarding legal requirements. All these characteristics depend on a specific application. For mechanical clean room production for instance in the field of microelectronics, electronics and mechanics, particles as solid matter are relevant, whereas for pharmatechnology, medical applications and chemistry, safety labs and food technology, microbiological impurities such as viruses, spores and fungus are relevant.

Material and personal flows have to be strictly separated in a clean room. Transferring materials into and out of a clean room is usually not very problematic, whereas the passage of persons into a clean room and out of a clean room may involve a lot of effort. This usually requires the user to change the entire clothing. However, this is cumbersome and involves a danger of introducing impurities. Maintenance, cleaning, and waste management as well as supply and equipment for such a person lock is expensive. It further requires a lot of time to introduce people into a clean room and to guide people out of such a clean room.

In view of these considerations, a clean room arrangement is provided according to an exemplary embodiment which can be constructed with a small ground area of for instance about 2.5 m² and a combined locking technology. The construction can be considered as some kind of air shower with gas blow processes. The basic construction may be a double wall construction with two mutually electrically lockable doors, a double bottom chamber with defined stand surfaces made of quartz glass and a ceiling have integrated therein members such as ventilators and filters. Left and right next to the doors, vertical air channels may be provided in which air jet nozzles may be integrated. In side walls, it is possible to integrate back flow openings and pre-filter units as well as decontamination, irradiation and/or spray systems may be integrated. For the cleaning of the clothing using a force being directed out of the clothing and for the implementation of the decontamination, bow shaped handle mechanisms may be implemented. A pressure air arrangement may be integrated with corresponding safety circuitry.

According to an exemplary embodiment it is possible to eliminate the multiple change of clothing by providing a clean room clothing system allowing to move between different clean room classes and safety zones without changing the clothes. Furthermore, a cross-contamination or undesired carry over of particles may be safely prevented. The lock ensures that on the one hand the clothing is cleaned and is decontaminated over its entire surface, and on the other hand provides safety against an uncontrolled access of persons. Regulation of various pressure levels can be ensured by the clean room lock as well.

Regarding the clean room clothing, a two layer textile system may be provided with an inner fabric portion and an outer fabric portion. Both fabric portions may be connected to one another and may be therefore integrally formed. The interior portion may be close to the body and may prevent am undesired carryover of particles from the body into the intermediate zone. The outer fabric may be repellent regarding particles. At a defined position of the outer fabric (hence, easily accessible by the user), a membrane with an electrical pickup may be integrated into the tissue. The end at the hands and feet may be matched with regard to gloves and shoes and may be gas-tight. The head piece of the fabric may be fixedly connected with the body clothing and may thus be integrally formed with it. Exposed portions of the face may be covered by goggles. The design of the clothing, the tissue, an optional coating of the tissue, shoes, goggles and the membrane technology are advantageous features of the clean room suit.

For enabling a person to enter from an unclean region to a clean region, the person may enter, already wearing the above-described clean room suit, the clean room lock. The opposing doors may be locked. The person stands at indicia or markers arranged in the bottom so that the legs are arranged in a desired position. Afterwards, the person may attach an air tube to the membrane valve of the clean room adapter. The person may grip with the hands hand grips arranged close to the ceiling, so that the arms are in an elongated and straddled position. By contact members and other kinds of sensors, a correct position of the feet, of the air tube attached to the clean room suit and a correct use of the hand grips can be ensured and can be a criteria which has to be fulfilled before a cleaning procedure is initiated, for instance before a valve is opened for providing pressurized air. The clean room suit is thereby ventilated or inflated so that particles are blown from an interior to an exterior of the overall. Consequently, these particles are blown away to leave a surface of the fabric. In this operation state, the clean room suit may look similar to a balloon and may be free of folds.

When certain sensor signals indicate a proper positioning of the person in the clean room lock, an air shower may be automatically activated and the clean room air streaming away from the clean room suit can be blown further away via channels with integrated nozzles (for instance arranged at four sides of side walls or corners, in a diagonal manner). The released particles can be sucked away and filtered close to the bottom of the cabin.

It is possible that the entire air circulation is configured as a recirculation system. After a defined air rinse phase (for instance 5 to 8 seconds) a disinfection or sterilization system (irradiation with UV-C or the like and/or application or spraying of chemicals, sterilization agent, etc.) can be activated. Germs and other biological particles on the clean room suit can be destroyed. Below the transparent bottom, UV-C irradiation units may be mounted which may sterilize bottom portions (soles) of the shoes.

Such a decontamination procedure is not dangerous for human beings. UV-C rays do not penetrate deeply into the body surface. Exposed skin portions of the face (for instance close to the eyes) may be protected by goggles. A safety feature may be provided in connection with the goggles. After the entire cleaning and decontamination process (for instance 10 to 20 seconds) the process may be switched off and the door may be released from the clean side.

Should the person be positioned apart from an expected position (regarding hands, feet, etc.) or should the person not use the air tube in a proper way, or if such conditions change during the cleaning cycle, the process can be terminated immediately and can be started again before allowing access of the person to a clean room. All parameters and conditions being relevant regarding safety and/or cleanness may be permanently monitored.

In a similar manner, it is possible to perform a similar cleaning procedure when transferring a person from the clean to the unclean side. This may for instance be advantageous in the light of a danger of carrying over germs from an interior to an exterior position (for instance in safety labs, stock breeding, etc.). In this scenario, the cleaning procedure may be performed in both directions, if desired. However, it is possible that the cleaning procedure is only performed in one direction or that different cleaning procedures are performed in the two directions, in dependence on the passing direction.

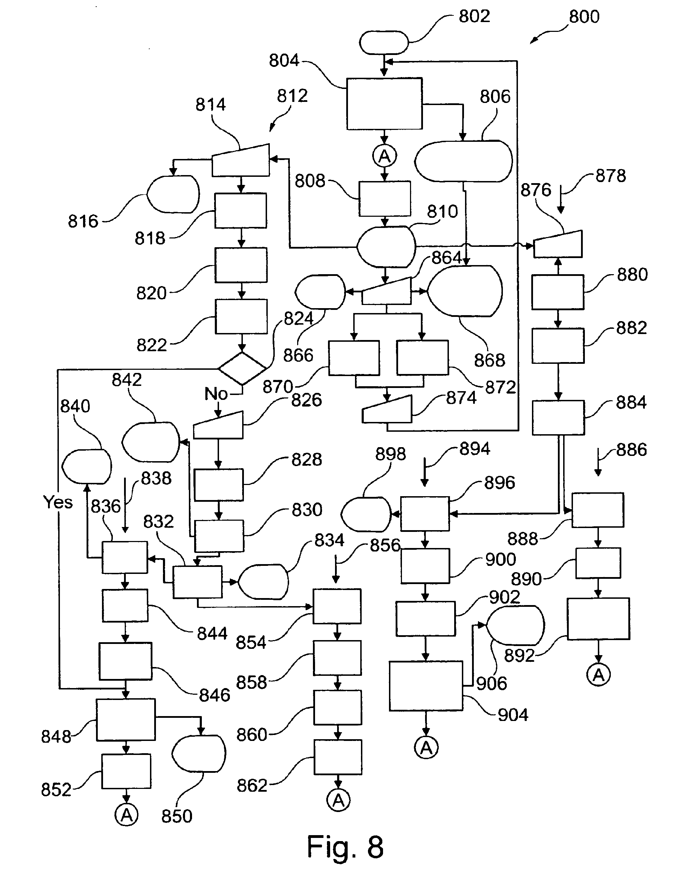

In the following, referring to **Fig. 8****,** a flow-chart 800 of operating the described systems will be explained.

Block 802 indicates switching on of a mains supply of the clean room system. In a block 804, a flushing procedure may be automatically started for a time of for instance 60 seconds, while both doors are locked. An LED (light-emitting diode) may be green at the clean and unclean sides, and the LED may blink green within the lock, as indicated by a block 806.

Both doors are unlocked in a block 808. Block 810 indicates that an LED at the clean side is green, as well as LEDs on the unclean side and in the lock.

Reference numeral 812 indicates an access to the clean room lock from the unclean side. In this scenario, a block 814 indicates that an entry via the door on the unclean side takes place. An LED on the clean side is green, whereas LEDs on the unclean side and in the lock are switched off. This is indicated by a block 816. In a block 818, the clean side door is locked. In a block 820, a person is within the lock and both doors are closed. In a block 822, the person is within the lock. Then a decision is made, see block 824 whether a time is larger than 15 seconds.

If no, an air flush is started, see block 826. Both doors are locked in a block 828. A flush time of 60 seconds is adjusted, see block 830. In block 832, both doors are unlocked. Block 834 indicates that an LED is green on the clean side, on the unclean side and within the lock.

Block 836 indicates that a door locking on the unclean side is unlocked and the person may exit via the unclean side. Therefore, reference numeral 838 indicates that the clean room lock is left via the unclean side.

Block 840 indicates that a green LED can be seen on the clean side, whereas the LED is switched off on the unclean side and in the lock.

A block 842 indicates that an LED blinks green at the clean side, and blinks green at the unclean side and within the lock.

In a block 844, the door on the clean side is locked. In a block 846, the door is left via the unclean side; afterwards, the door on the unclear side is closed. A block 848 is subsequently reached, and is also reached if the decision in block 824 is yes.

In block 848, an automatic flushing procedure is started with a flushing time of for instance 60 seconds. As indicated by a block 850, the LEDs blink green at the clean, unclean and interior side. Both doors are locked in a block 852, and the procedure continues with the block A.

If the clean room lock is left via the clean side, see reference numeral 856, the procedure continues with the block 854. In block 854, the door lock of the clean side is open and the person may exit via the clean side. In a block 858, the clean door is locked and in a block 860 the unclean door is locked. In a block 862, the door is left via a clean side, afterwards the clean door is closed. The procedure then continues with block A.

Coming back to block 810, a block 864 illustrates a scenario in which an emergency button is operated. As can be taken from a block 866, the LED on the clean side is green, and the LED is switched off on the unclean side and in the interior. In a block 868, the LEDs are red at the clean side, at the unclean side and in the interior, when emergency off has been pressed. As can be taken from a block 870, the locked door on the unclean side is unlocked, and, see block 872, the lock on the clean side is unlocked. The emergency button may be reset in a block 874.

Again coming back to block 810, now referring to an access to the lock from the clean side, compare reference numeral 878, whether there is an access to the door from the clean side. Block 880 corresponds to a locking of the unclean door. When the person is in the lock, both doors are closed, see block 882. Block 884 relates to both doors being locked.

Again, two scenarios can be distinguished. In a scenario denoted with reference numeral 886 (lock is left to the clean side), the door is opened in a block 888 and the unclean door is locked in a block 890. Furthermore, in a block 892, the person leaves the clean door, after this the clean door clean. The procedure then continues with block A.

In an alternative scenario after block 884, the lock may be left via the unclean side, see reference numeral 894. The door on the unclean side may be opened, see block 896, and the LED may be green on the clean side, and may be off at the unclean side and the interior of the lock, see block 898. The clean door may be locked, see block 900. When block 902 corresponds to a person leaving the lock via the unclean door, afterwards the unclean door is closed. A block 904 automatically starts flushing for a flushing time of for instance 60 seconds, and both doors are locked. The procedure then continues with block A.

Block 906 indicates that the LED blinks green on the clean side, the unclean side and in an interior of the lock.

It should be noted that the term "comprising" does not exclude other elements or features and the "a" or "an" does not exclude a plurality. Also elements described in association with different embodiments may be combined.

It should also be noted that reference signs in the claims shall not be construed as limiting the scope of the claims.

## Claims

1. A clean room suit 100 for clothing a person in a clean room, the clean room suit comprising
a fabric 102 configured to cover at least a part of a body of the person, the fabric comprises an inner fabric portion, particularly a lining portion, to be placed directly on at least the part of the body and comprises an outer fabric portion, particularly a shell fabric portion, to be exposed to an environment of the clean room suit 100; and
a clean room lock adapter 104 arranged on and/or in the fabric 102 and clean lock being configured for coupling to a clean room (150) lock in such a manner that, in an operation mode in which the clean room lock is coupled to the clean room lock adapted 104 a cleaning procedure is activated, particles on and/or in the fabric are moved out of the fabric towards an exterior of the clean room suit (100)

2. The clean room suit of claim 1, wherein the inner fabric portion and the outer fabric portion are connected to one another.

3. The clean room suit of claim 1 or 2, wherein the clean room lock adapter is arranged on and/or in the outer fabric portion.

4. The clean room suit of any one of claims 1 to 3, wherein the inner fabric portion has at least one property of the group consisting of breathable, eudermic, and impermeable for particles from the body of the person, and wherein the outer fabric portion has at least one property of the group consisting of repellant against particles, and clean room compatible.

5. The clean room suit of any one of claims 1 to 4, wherein the clean room lock adapter comprises a connection flange configured for mounting on a correspondingly configured connection flange of the clean room lock.

6. The clean room suit of any one of claims 1 to 5, wherein the clean room lock adapter is configured for blowing a fluid, particularly a gas, supplied by the clean room lock to an interior of the fabric through the fabric towards an outer surrounding of the fabric so that the particles on and/or in the fabric are moved out of the fabric towards the exterior of the clean room suit.

7. The clean room suit of any one of claims 1 to 6, wherein the fabric, particularly the outer portion of the fabric, comprises one or more antimicrobial agents being capable of controlling microbial growth on clean room equipment, wherein the one or more antimicrobial agents are of organic origin.

8. A clean room lock 150 for cleaning a person transmitting between an interior and an exterior of a clean room, the clean room lock 150 comprising
a clean room suit adapter 152 configured for coupling to a clean room suit 100 to clothe the person and configured so that, in an operation mode in which the clean room suit adapter (152) is coupled to the clean room suit 100 and a cleaning procedure is activated, particles on and/or in the clean room suit (100) are moved out of the clean room suit 100 towards an exterior of the clean room suit 100, wherein the clean room suit adapter 152 comprises a connection flange configured for mounting on a correspondingly configured connection flange of the clean room suit (100).

9. The clean room lock of claim 8, wherein the clean room suit adapter is configured for providing a pressurized fluid, particularly a gas, to the clean room suit so that the particles on and/or in the clean room suit are moved out of the clean room suit towards the exterior of the clean room suit.

10. The clean room lock of claim 8 or 9, further comprising an ultraviolet radiation source configured for irradiating the clean room suit with ultraviolet radiation, particularly comprising an ultraviolet radiation source accommodated below the bottom wall of the clean room lock, the bottom wall particularly comprising a transparent material, and configured for irradiating the clean room suit from below with ultraviolet radiation.

11. The clean room lock of any one of claims 8 to 10, further comprising a gas jet unit, particularly an air jet unit, the gas jet unit comprising a plurality of nozzles, each adapted for blowing a gas around an exterior of the clean room suit, wherein the plurality of nozzles are arranged in a plurality of groups, wherein nozzles of the same group are arranged along a vertical row and each blow along a same direction, and nozzles of different groups are arranged to blow along different directions.

12. The clean room lock of any one of claims 8 to 11, further comprising a decontamination unit, particularly a chemical decontamination unit, adapted for ejecting a decontamination agent, particularly a chemical agent, for decontaminating the clean room suit.

13. The clean room lock of any of claims 8 to 12, further comprising a control unit adapted for automatically executing a predefined cleaning procedure , wherein the control unit is configured for automatically executing the predefined cleaning procedure only upon detecting that simultaneously:
- the clean room suit adapter is coupled to the clean room suit;
- feet of the person stand at a target position;
- hands of the person grip a handle.

14. A clean room arrangement, comprising
a clean room suit 100 as defined in any one of claims 1 to 7; and
a clean room lock 150 as defined in any one of claims 8 to 13;
wherein the clean room suit adapter and the clean room lock adapter are configured for coupling to one another.

15. A method of managing a transition of a person in a clean room lock, particularly a clean room lock as defined in any one of claims 8 to 13, between an interior and an exterior of a clean room, the method comprising
clothing the person with a clean room suit having a fabric covering at least a part of a body of the person, particularly a clean room suit as defined in any one of claims 1 to 7;
coupling a clean room lock adapter arranged on and/or in the fabric of the clean room suit to a clean room suit adapter of the clean room lock;
activating a cleaning procedure controlled by the clean room lock so that particles on and/or in the fabric are moved out of the fabric towards an exterior of the clean room suit.

## Patentansprüche

1. Ein Reinraumanzug (100) zum Kleiden einer Person in einem Reinraum, wobei der Reinraumanzug aufweist
einen Stoff (102), welcher konfiguriert ist, zumindest einen Teil eines Körpers der Person zu bedecken, wobei der Stoff einen inneren Stoffabschnitt aufweist, insbesondere einen Innenfutterabschnitt, um direkt auf zumindest dem Teil des Körpers platziert zu werden, und einen äußeren Stoffabschnitt aufweist, insbesondere einen Hüllenstoffabschnitt, um einer Umgebung des Reinraumanzugs (100) ausgesetzt zu werden, und
einen Reinraumschleusenadapter (104), welcher auf und/oder in dem Stoff (102) angeordnet ist und zum Koppeln an eine Reinraumschleuse (150) in solch einer Art und Weise konfiguriert ist, dass in einem Betriebsmodus, in welchem die Reinraumschleuse an dem Reinraumschleusenadapter (104) gekoppelt ist und ein Reinigungsprozess aktiviert ist, Partikel auf und/oder in dem Stoff in Richtung eines Äußeren des Reinraumanzugs (100) aus dem Stoff bewegt werden.

2. Der Reinraumanzug gemäß Anspruch 1, wobei der innere Stoffabschnitt und der äußere Stoffabschnitt miteinander verbunden sind.

3. Der Reinraumanzug gemäß Anspruch 1 oder 2, wobei der Reinraumschleusenadapter auf und/oder in dem äußeren Stoffabschnitt angeordnet ist.

4. Der Reinraumanzug gemäß irgendeinem der Ansprüche 1 bis 3, wobei der innere Stoffabschnitt zumindest eine Eigenschaft der Gruppe bestehend aus atmungsaktiv, hautfreundlich und undurchlässig für Partikel von dem Körper der Person hat, und wobei der äußere Stoffabschnitt zumindest eine Eigenschaft aus der Gruppe bestehend aus abweisend gegen Partikel und reinraumkompatibel hat.

5. Der Reinraumanzug gemäß irgendeinem der Ansprüche 1 bis 4, wobei der Reinraumschleusenadapter einen Verbindungsflansch aufweist, welcher zum Befestigen an einem korrespondierend konfigurierten Verbindungsflansch der Reinraumschleuse konfiguriert ist.

6. Der Reinraumanzug gemäß irgendeinem der Ansprüche 1 bis 5, wobei der Reinraumschleusenadapter zum Blasen eines Fluids, insbesondere eines Gases, durch den Stoff hindurch in Richtung einer äußeren Umgebung des Stoffes konfiguriert ist, welches mittels der Reinraumschleuse an einem Inneren des Stoffs bereitgestellt ist, so dass die Partikel auf und/oder in dem Stoff in Richtung des Äußeren des Reinraumanzugs aus dem Stoff bewegt werden.

7. Ein Reinraumanzug gemäß irgendeinem der Ansprüche 1 bis 6, wobei der Stoff, insbesondere der äußere Abschnitt des Stoffs, ein oder mehr antimikrobielle Mittel aufweist, welche zur Steuerung eines mikrobiellen Wachstums auf einer Reinraumausrüstung fähig sind, wobei das eine oder die mehreren antimikrobiellen Mittel organischen Ursprungs ist oder sind.

8. Eine Reinraumschleuse (150) zum Reinigen einer Person, welche zwischen einem Inneren und einem Äußeren eines Reinraums herüber geleitet wird, wobei die Reinraumschleuse (150) aufweist
einen Reinraumanzugadapter (152), welcher konfiguriert ist zum Koppeln an einen Reinraumanzug (100), um die Person zu kleiden, und konfiguriert ist, so dass, in einem Betriebsmodus, in welchem der Reinraumanzugadapter (152) an den Reinraumanzug (100) gekoppelt ist und ein Reinigungsprozess aktiviert ist, Partikel auf und/oder in dem Reinraumanzug (100) in Richtung eines Äußeren des Reinraumanzugs (100)aus dem Reinraumanzug (100) bewegt werden, wobei der Reinraumanzugadapter (152) einen Verbindungsflansch aufweist, welcher zum Befestigen an einem korrespondierend konfigurierten Verbindungsflansch des Reinraumanzugs (100) konfiguriert ist.

9. Die Reinraumschleuse gemäß Anspruch 8, wobei der Reinraumanzugadapter zum Bereitstellen eines druckbeaufschlagten Fluids, insbesondere eines Gases, an den Reinraumanzug konfiguriert ist, so dass die Partikel auf und/oder in dem Reinraumanzug in Richtung des Äußeren des Reinraumanzugs aus dem Reinraumanzug bewegt werden.

10. Die Reinraumschleuse gemäß Anspruch 8 oder 9, welche ferner eine ultraviolette Strahlungsquelle aufweist, welche zum Bestrahlen des Reinraumanzugs mit ultravioletter Strahlung konfiguriert ist, insbesondere eine ultraviolette Strahlungsquelle aufweisend, welche unterhalb der Bodenwand der Reinraumschleuse angebracht ist, wobei die untere Wand insbesondere ein transparentes Material aufweist, und konfiguriert ist zum Bestrahlen des Reinraumanzugs von unten mit ultravioletter Strahlung.

11. Die Reinraumschleuse gemäß irgendeinem der Ansprüche 8 bis 10, welche ferner eine Drucklufteinheit aufweist, insbesondere eine Drucklufteinheit, wobei die Drucklufteinheit eine Mehrzahl von Düsen aufweist, welche jeweils zum Blasen eines Gases um ein Äußeres des Reinraumanzugs herum angepasst sind, wobei die Mehrzahl der Düsen in einer Mehrzahl von Gruppen eingerichtet sind, wobei die Düsen der gleichen Gruppe entlang einer vertikalen Reihe eingerichtet sind und jede entlang einer gleichen Richtung bläst, und die Düsen von unterschiedlichen Gruppen eingerichtet sind, entlang unterschiedlicher Richtungen zu blasen.

12. Die Reinraumschleuse gemäß irgendeinem der Ansprüche 8 bis 11, welche ferner eine Dekontaminationseinheit aufweist, insbesondere eine chemische Dekontaminationseinheit, welche zum Auswerfen eines Dekontaminationsmittels, insbesondere eines chemischen Mittels, zum Dekontaminieren des Reinraumanzugs angepasst ist.

13. Die Reinraumschleuse gemäß irgendeinem der Ansprüche 8 bis 12, welche ferner eine Steuereinheit aufweist, welche zum automatischen Ausführen eines vordefinierten Reinigungsprozesses angepasst ist, wobei die Steuereinheit zum automatischen Ausführen des vordefinierten Reinigungsprozesses nur nach einem Detektieren konfiguriert ist, dass gleichzeitig:
- der Reinraumanzugadapter an den Reinraumanzug gekoppelt ist,
- die Füße der Person auf einer Zielposition stehen,
- die Hände der Person einen Handgriff greifen.

14. Eine Reinraumanordnung, welche aufweist
einen Reinraumanzug (100) wie in irgendeinem der Ansprüche 1 bis 7 definiert, und
eine Reinraumschleuse (150) wie in irgendeinem der Ansprüche 8 bis 13 definiert,
wobei der Reinraumanzugadapter und der Reinraumschleusenadapter zum miteinander Koppeln konfiguriert sind.

15. Ein Verfahren zum Managen eines Herüberleitens einer Person in einer Reinraumschleuse, insbesondere eine Reinraumschleuse wie in irgendeinem der Ansprüche 8 bis 13 definiert, zwischen einem Inneren und einem Äußeren eines Reinraums, wobei das Verfahren aufweist
Kleiden der Person mit einem Reinraumanzug, welcher einen Stoff hat, welcher zumindest einen Teil eines Körpers der Person bedeckt, insbesondere einen Reinraumanzug wie in irgendeinem der Ansprüche 1 bis 7 definiert,
Koppeln eines Reinraumschleusenadapters, welcher auf und/oder in dem Stoff des Reinraumanzugs angeordnet ist, an einen Reinraumanzugadapter der Reinraumschleuse,
Aktivieren eines Reinigungsprozesses, welcher mittels der Reinraumschleuse gesteuert wird, so dass Partikel auf und/oder in dem Stoff in Richtung eines Äußeren des Reinraumanzugs aus dem Stoff bewegt werden.

## Revendications

1. Combinaison pour salle blanche 100 destinée à habiller une personne dans une salle blanche, la combinaison pour salle blanche comprenant
un tissu 102 configuré pour couvrir au moins une partie du corps d'une personne, le tissu comprend une partie de tissu interne, en particulier une partie de doublure, à placer directement sur au moins la partie du corps et comprend une partie de tissu externe, en particulier une partie de tissu enveloppe, à exposer à l'environnement de la combinaison pour salle blanche 100 ; et
un adaptateur de sas de salle blanche 104 disposé sur et/ou dans le tissu 102 et configuré pour le couplage à un sas de salle blanche 150 de telle manière que, dans un mode de fonctionnement dans lequel le sas de salle blanche est couplé à l'adaptateur de sas de salle blanche 104 et une procédure de nettoyage est activée, des particules sur et/ou dans le tissu sont déplacées du tissu vers l'extérieur de la combinaison pour salle blanche 100.

2. Combinaison pour salle blanche selon la revendication 1, dans laquelle la partie de tissu interne et la partie de tissu externe sont reliées l'une à l'autre.

3. Combinaison pour salle blanche selon la revendication 1 ou 2, dans laquelle l'adaptateur de sas de salle blanche est disposé sur et/ou dans la partie de tissu externe.

4. Combinaison pour salle blanche selon l'une quelconque des revendications 1 à 3, dans laquelle la partie de tissu interne présente au moins une propriété du groupe se composant de respirante, eudermique, et imperméable aux particules du corps de la personne, et dans laquelle la partie de tissu externe présente au moins une propriété du groupe se composant de répulsive aux particules, et compatible avec la salle blanche.

5. Combinaison pour salle blanche selon l'une quelconque des revendications 1 à 4, dans laquelle l'adaptateur de sas de salle blanche comprend une bride de liaison configurée pour le montage sur une bride de liaison configurée de manière correspondante du sas de salle blanche.

6. Combinaison pour salle blanche selon l'une quelconque des revendications 1 à 5, dans laquelle l'adaptateur de sas de salle blanche est configuré pour souffler un fluide, en particulier un gaz, fourni par le sas de salle blanche à l'intérieur du tissu au travers du tissu vers un entourage externe du tissu de sorte que les particules sur et/ou dans le tissu soient retirées du tissu vers l'extérieur de la combinaison pour salle blanche.

7. Combinaison pour salle blanche selon l'une quelconque des revendications 1 à 6, dans laquelle le tissu, en particulier la partie extérieure du tissu, comprend un ou plusieurs agents antimicrobiens capables de contrôler la prolifération microbienne sur l'équipement de salle blanche, dans laquelle un ou plusieurs agents antimicrobiens sont d'origine organique.

8. Sas de salle blanche 150 destiné au nettoyage d'une personne transitant entre un intérieur et un extérieur d'une salle blanche, le sas de salle blanche 150 comprenant
un adaptateur 152 de combinaison pour salle blanche configuré pour le couplage avec une combinaison pour salle blanche 100 destinée à habiller la personne et configuré de sorte que, dans un mode de fonctionnement dans lequel l'adaptateur de combinaison pour salle blanche 152 est couplé à la combinaison pour salle blanche 100 et une procédure de nettoyage est activée, des particules sur et/ou dans la combinaison pour salle blanche 100 soient déplacées hors de la combinaison pour salle blanche 100 vers l'extérieur de la combinaison pour salle blanche 100, dans lequel l'adaptateur de combinaison pour salle blanche 152 comprend une bride de liaison configurée pour le montage sur une bride de liaison de la combinaison pour salle blanche 100 configurée de manière correspondante.

9. Sas de salle blanche selon la revendication 8, dans lequel l'adaptateur de combinaison pour salle blanche est configuré pour fournir un fluide sous pression, en particulier du gaz, à la combinaison pour salle blanche de sorte que les particules sur et/ou dans la combinaison pour salle blanche soient déplacées hors de la combinaison pour salle blanche vers l'extérieur de la combinaison pour salle blanche.

10. Sas de salle blanche selon la revendication 8 ou 9, comprenant en outre une source de rayonnement ultraviolet configurée pour irradier la combinaison pour salle blanche avec un rayonnement ultraviolet, comprenant en particulier une source de rayonnement ultraviolet logée sous la paroi inférieure du sas de salle blanche, la paroi inférieure comprenant en particulier un matériau transparent, et configurée pour irradier la combinaison pour salle blanche depuis le dessous avec un rayonnement ultraviolet.

11. Sas de salle blanche selon l'une quelconque des revendications 8 à 10, comprenant en outre une unité à jet de gaz, en particulier une unité à jet d'air, l'unité à jet de gaz comprenant une pluralité de buses, chacune adaptée pour souffler un gaz autour de l'extérieur de la combinaison pour salle blanche, dans lequel la pluralité de buses est disposée dans une pluralité de groupes, dans lequel des buses du même groupe sont disposées le long d'une rangée verticale et soufflent chacune dans une même direction, et des buses de différents groupes sont disposées pour souffler dans différentes directions.

12. Sas de salle blanche selon l'une quelconque des revendications 8 à 11, comprenant en outre une unité de décontamination, en particulier une unité de décontamination chimique, adaptée pour éjecter un agent de décontamination, en particulier un agent chimique, pour décontaminer la combinaison pour salle blanche.

13. Sas de salle blanche selon l'une quelconque des revendications 8 à 12, comprenant en outre une unité de commande adaptée pour exécuter automatiquement une procédure de nettoyage prédéfinie, dans lequel l'unité de commande est configurée pour exécuter automatiquement la procédure de nettoyage prédéfinie seulement après avoir détecté simultanément que :
- l'adaptateur de combinaison pour salle blanche est couplé à la combinaison pour salle blanche ;
- les pieds de la personne se tiennent sur une position cible ;
- les mains de la personne saisissent un manche.

14. Ensemble de salle blanche comprenant
une combinaison pour salle blanche 100 selon l'une quelconque des revendications 1 à 7 ; et
un sas de salle blanche 150 selon l'une quelconque des revendications 8 à 13 ;
dans lequel l'adaptateur de combinaison pour salle blanche et l'adaptateur de sas de salle blanche sont configurés pour être couplés l'un à l'autre.

15. Procédé de gestion d'une transition d'une personne dans un sas de salle blanche, en particulier un sas de salle blanche selon l'une quelconque des revendications 8 à 13, entre un intérieur et un extérieur d'une salle blanche, le procédé comprenant
l'habillage de la personne avec une combinaison pour salle blanche ayant un tissu couvrant au moins une partie d'un corps de la personne, en particulier une combinaison pour salle blanche selon l'une quelconque des revendications 1 à 7 ;
le couplage d'un adaptateur de sas de salle blanche disposé sur et/ou dans le tissu de la combinaison pour salle blanche avec un adaptateur de combinaison pour salle blanche du sas de salle blanche ;
l'activation d'une procédure de nettoyage commandée par le sas de salle blanche de sorte que des particules sur et/ou dans le tissu soient déplacées hors du tissu vers un extérieur de la combinaison de salle blanche.
